# EUROPEAN PATENT SPECIFICATION

(11) **EP 2 603 605 B1**
(45) Date of publication and mention of the grant of the patent: **21.01.2015**
(21) Application number: 11743078.5
(22) Date of filing: 12.08.2011
(51) Int. Cl.: C12Q 1/68

(54) **A METHOD FOR CLASSIFYING AN INFLAMMATORY BOWEL DISEASE AS A CROHN'S DISEASE OR AS AN ULCERATIVE COLITIS**
VERFAHREN ZUR KLASSIFIZIERUNG EINER ENTZÜNDLICHEN DARMERKRANKUNG WIE MORBUS CROHN ODER COLITIS ULCEROSA
MÉTHODE DE CLASSIFICATION D'UNE AFFECTION INTESTINALE INFLAMMATOIRE NON SPÉCIFIQUE DANS LA CATÉGORIE DE LA MALADIE DE CROHN OU DE LA RECTOCOLITE HÉMORRAGIQUE

(30) Priority: 13.08.2010 EP 10305889
(43) Date of publication of application: 19.06.2013
(73) Proprietor: INSERM (Institut National de la Santé et de la Recherche Médicale), 75013 Paris (FR); Université Paris Diderot - Paris 7, 75013 Paris (FR)
(72) Inventor: OGIER-DENIS, Eric, F-75018 Paris (FR); TRETON, Xavier, F-75018 Paris (FR)
(74) Representative: Cabinet Plasseraud
(86) International application number: PCT/EP2011/063949
(87) International publication number: WO 2012/020126

(56) References cited:
- WO-A2-2009/120877
- X. TRETON*, M. FASSEU, E. PEDRUZZI, Y. BOUHNIK, T. APARICIO, D. CAZALS-HATEM, F. DANIEL, M. LABURTHE, A. GROYER, E. OGIER-DENIS: "P0273: ALTERATION OF MICRORNA EXPRESSION IN INFLAMED AND INACTIVE COLONIC MUCOSA OF PATIENTS WITH INFLAMMATORY BOWEL DISEASE", GASTRO 2009 - UEGW/WCOGH, LONDON, 20 November 2009 (2009-11-20), - 25 November 2009 (2009-11-25), XP002605580,
- TRETON ET AL: "P217 SPECIFIC ALTERATION OF MICRO-RNA EXPRESSION IN INFLAMMATORY BOWEL DISEASE", JOURNAL OF CROHN'S AND COLITIS, vol. 2, no. 1, 1 February 2008 (2008-02-01), page S69, XP022522516,
- Treton et al.: "Altération de l'expression des microARN dans la muqueuse colique quiescente de patients atteints de maladies inflammatoires chroniques de l'intestin (MICI)", , 26 March 2010 (2010-03-26), XP002605581, Retrieved from the Internet: URL:http://www.snfge.org/data/ModuleMiseEn Ligne/HTML/Resumes/4859.html [retrieved on 2010-10-15]
- WU F ET AL: "MicroRNAs Are Differentially Expressed in Ulcerative Colitis and Alter Expression of Macrophage Inflammatory Peptide-2alpha", GASTROENTEROLOGY,, vol. 135, no. 5, E24, 1 November 2008 (2008-11-01), pages 1624-1635, XP025624064, ISSN: 0016-5085, DOI: 10.1053/J.GASTRO.2008.07.068 [retrieved on 2008-08-03] cited in the application

## Description

### FIELD OF THE INVENTION:

The present invention relates to a method for classifying an inflammatory bowel disease in a patient as a Crohn's disease or as an ulcerative colitis.

### BACKGROUND OF THE INVENTION:

Inflammatory bowel diseases (IBD), such as Crohn's disease (CD) and ulcerative colitis (UC) are severe and relapsing immunologically mediated chronic disorders of the gastrointestinal tract. IBDs are heterogeneous diseases characterized by various genetic abnormalities that lead to overly aggressive inflammatory responses to a subset of commensal enteric bacteria.

Crohn's disease can affect all parts of the digestive tract and specially the ileum and/or colon and leads to mucosal ulcerations, fistula, and deep infiltration of inflammatory cells in the bowel wall.

Ulcerative colitis often involves the lower part of the colon and the rectum and mucosal inflammation may extend to the caecum in a contiguous pattern.

In clinical practice, 20 to 30% of patients with IBD colitis cannot be classified as CD or UC based upon usual endoscopic, radiologic, and histopathologic criteria, though this distinction may be crucial to guide therapeutic choices, especially when colonic resection is discussed.

Similarly, the sensitivity of serological markers (autoantibodies to neutrophils [ANCA, pANCA] and antimicrobial antibodies [ASCA, anti-OmpC, anti-I2, and anti-CBir1]) remains insufficient to discriminate between CD and UC. On the other hand, the aetiology of IBDs and the cause of flare still remain largely unknown and specific biomarkers of CD and UC are also needed to assess an early diagnosis. To date, strictly specific biomarkers remain difficult to elect.

Some specific signatures of miRNAs expression in the colonic mucosa of patients with IBD were already disclosed in the prior art (X. Treton*, M. Fasseu, E. Pedruzzi, Y. Bouhnik, T. Aparicio, D. Cazals-Hatem, F. Daniel, M. Laburthe, A. Grover, E. Ogierdenis: "P0273: Alteration Of Microrna Expression In Inflamed And Inactive Colonic Mucosa Of Patients With Inflammatory Bowel Disease", Gastro 2009- Uegw/Wcogh, London, 20 November 2009 (2009-11-20), - 25 November 2009 (2009-11-25); TRETON ET AL: "P217 SPECIFIC ALTERATION OF MICRO-RNA EXPRESSION IN INFLAMMATORY BOWEL DISEASE", JOURNAL OF CROHN'S AND COLITIS, vol. 2, no. 1, 1 February 2008 (2008-02-01), page S69; Treton et al.: "Alteration de l'expression des microARN dans la muqueuse colique quiescente de patients atteints de maladies inflammatoires chroniques de l'intestin (MICI)", 26 March 2010 (2010-03-26); and WO 2009/120877). However said signatures do not allow at the same time the diagnosis of IBD (IBD vs healthy) and the differential diagnosis of UC and CD.

### SUMMARY OF THE INVENTION:

The present invention relates to a method for classifying an inflammatory bowel disease in a patient as a CD or as an UC, said method comprising:
i) a step of measuring an expression profile of miRNAs in a sample isolated in non-inflamed mucosa of the patient's colon, wherein said miRNAs are miR15a, miR26a, miR29a, miR29b, miR30c, miR126*, miR127-3p, miR-142-3p, miR-142-5p, miR-146a, miR-146b-5p, miR150, miR-181d, miR-182, miR185, miR196a, miR199a-3p, miR199a-5p, miR199b-5p, miR-203, miR223, miR-299-5p, miR320a, miR324-3p, and miR-328 and
ii) a step consisting of comparing the expression profile of said miRNAs in the sample of the patient with control profiles of said miRNAs, wherein a difference or similarity between said expression profiles is indicative of a Crohn's disease or an ulcerative colitis.

### DETAILED DESCRIPTION OF THE INVENTION:

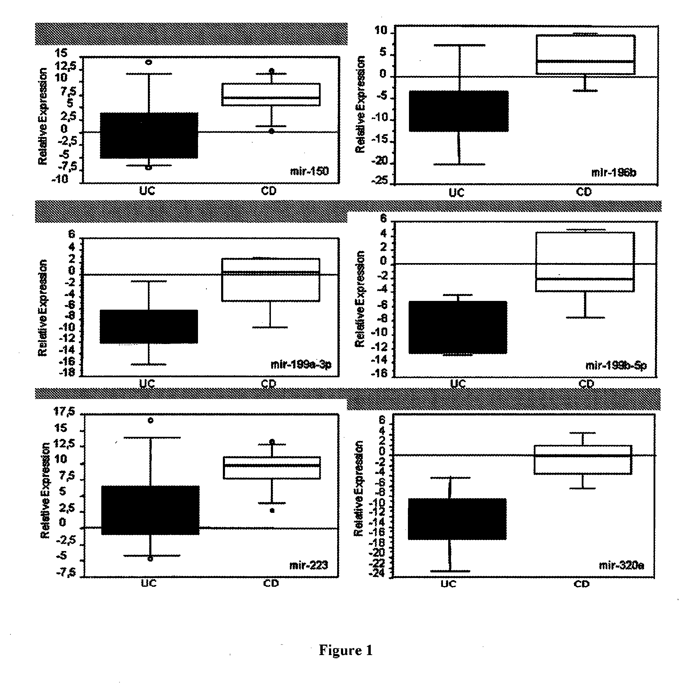

The inventors' objective was to pinpoint alterations of miRNA gene expression in the quiescent, non-inflamed colonic mucosa of IBD patients *vs.* that of healthy individuals. Accordingly, they have studied genome-wide alterations in the pattern of miRNA gene expression in quiescent mucosa of patients with active UC and CD, in comparison with normal healthy tissue. They have identified subsets of 22 (UC) and 34 (CD) miRNAs with highly altered expression in quiescent mucosal tissue, 10 being commonly dysregulated in non-inflamed UC and CD tissues (Table B). More particularly the inventors selected 15 miRNAs for which the expression was statistically different in non inflamed colonic biopsises of UC and CD patients (see Table B and Figure 1). These miRNAs and the 10 being commonly dysregulated in non-inflamed UC and CD tissues were then tested for their ability to discriminate between CD and UC. Based on the clinical classification of the panel of patients as UC or CD, the selection of the 25 miRNAs was able to predictl5/16 patients in their true class (Table V).

Accordingly, the present invention relates to a method for classifying an inflammatory bowel disease in a patient as a Crohn's disease or as an ulcerative colitis, said method comprising a step of measuring an expression profile of miRNAs in a sample from the patient, wherein said miRNAs are miR15a, miR26a, miR29a, miR29b, miR30c, miR126*, miR127-3p, miR-142-3p, miR-142-5p, miR-146a, miR-146b-5p, miR150, miR-181d, miR-182, miR185, miR196a, miR199a-3p, miR199a-5p, miR199b-5p, miR-203, miR223, miR-299-5p, miR320a, miR324-3p, and miR-328.

The term "miRNAs" refers to mature microRNA (non-coding small RNAs) molecules that are generally 21 to 22 nucleotides in length, even though lengths of 19 and up to 23 nucleotides have been reported. miRNAs are each processed from longer precursor RNA molecules ("precursor miRNA": pri-miRNA and pre-miRNA). Pri-miRNAs are transcribed either from non-protein-encoding genes or embedded into protein-coding genes (within introns or non-coding exons). The "precursor miRNAs" fold into hairpin structures containing imperfectly base-paired stems and are processed in two steps, catalyzed in animals by two Ribonuclease III-type endonucleases called Drosha and Dicer. The processed miRNA is typically a portion of the stem. The processed miRNAs (also referred to as "mature miRNA") are assembled into large ribonucleoprotein complexes (miRISCs) that post-transcriptional repression (down-regulation) of a specific target gene(s).

All the miRNAs pertaining to the invention are known *per se* and sequences of them are publicly available from the data base http://www.mirbase.org/cgi-bin/mirna summary.pl?org=hsa. The miRNAs of the invention are listed in Table A:

**TABLE A: miRNAs pertaining to the invention**

| **miRNA**_**Id** | **Accession_Number** | **Sequence** | **SEQ ID NO:** |
|---|---|---|---|
| hsa-mir-15a | MIMAT0000068 | uagcagcacauaaugguuugug | 1 |
| hsa-mir-26a | MIMAT0000082 | uucaaguaauccaggauaggcu | 2 |
| hsa-mir-29a | MIMAT0000086 | uagcaccaucugaaaucgguua | 3 |
| hsa-mir-29b | MIMAT0000100 | uagcaccauuugaaaucaguguu | 4 |
| hsa-mir-30c | MIMAT0000244 | uguaaacauccuacacucucagc | 5 |
| hsa-mir-126* | MIMAT0000444 | cauuauuacuuuugguacgcg | 6 |
| hsa-mir-127-3p | MIMAT0000446 | ucggauccgucugagcuuggcu | 7 |
| hsa-mir-146a | MIMAT0000449 | ugagaacugaauuccauggguu | 8 |
| hsa-mir-150 | MIMAT0000451 | ucucccaacccuuguaccagug | 9 |
| hsa-mir-142-5p | MIMAT0000433 | cauaaaguagaaagcacuacu | 10 |
| hsa-mir-142-3p | MIMAT0000434 | uguaguguuuccuacuuuaugga | 11 |
| hsa-mir-146b-5p | MIMAT0002809 | ugagaacugaauuccauaggcu | 12 |
| hsa-mir-181d | MIMAT0002821 | aacauucauuguugucggugggu | 13 |
| hsa-mir-182 | MIMAT0000259 | uuuggcaaugguagaacucacacu | 14 |
| hsa-mir-185 | MIMAT0000455 | uggagagaaaggcaguuccuga | 15 |
| hsa-mir-196a | MIMAT0000226 | uagguaguuucauguuguuggg | 16 |
| hsa-mir-199a-3p | MIMAT0000232 | acaguagucugcacauugguua | 17 |
| hsa-mir-199a-5p | MIMAT0000231 | cccaguguucagacuaccuguuc | 18 |
| hsa-mir-199b-5p | MIMAT0000263 | cccaguguuuagacuaucuguuc | 19 |
| hsa-mir-203 | MIMAT0000264 | gugaaauguuuaggaccacuag | 20 |
| hsa-mir-223 | MIMAT0000280 | ugucaguuugucaaauacccca | 21 |
| hsa-mir-299-5p | MIMAT0002890 | ugguuuaccgucccacauacau | 22 |
| hsa-mir-320 | MIMAT0000510 | aaaagcuggguugagagggcga | 23 |
| hsa-mir-324-3p | MIMAT0000762 | acugccccaggugcugcugg | 24 |
| hsa-mir-328 | MIMAT0000752 | cuggcccucucugcccuuccgu | 25 |

Among the 25 miRNAs of Table 5, 10 were identified as commonly dysregulated in non-inflamed UC and CD tissues and 15 were for which the expression was statistically different in non inflamed colonic biopsises of UC and CD patients (see Table B and Figure 1). Accordingly, the 10 miRNAs of the profile commonly dysregulated in non-inflamed UC and CD tissues allow the confirmation that the patient is affected with an inflammatory disease and the remaining 15 ones are used to discriminate between UC and CD.

**Table B: selection of the 25 miRNAs**

| | |
|---|---|
| **miRNAs commonly dysregulated in non-inflamed UC and CD tissues** | miR-15a, miR-26a, miR-29a, miR-29b, miR-30c, miR-126*, miR-127-3p, miR-185, miR-196a, miR-324-3p |
| **miRNAs that discriminate between UC and CD** | miR-146b-5p, miR150, miR-181d, miR-182, miR199a-3p, miR199a-5p, miR199b-5p, miR-203, miR223, miR-299-5p, miR320a, miR-146a, miR-142-3p, miR-142-5p, miR-328 |

The term "miRNA profile" refers to a set of data regarding the expression pattern for the miRNAs from Table A in the sample.

The term "sample" means any sample derived from the colon of the patient, which comprise mucosal cells. Said sample is obtained for the purpose of *the in vitro* evaluation. In a particular embodiment the sample results from an endoscopical biopsy performed in the colon of the patient. Said endoscopical biopsy may be taken from various areas of the colon. In another particular embodiment, the sample may be isolated from non-inflamed mucosa of the patient's colon. Consequently, the invasivness of the method according to the invention is relatively limited without the need of anesthetizing the patient or of purging the patient's intestines.

In another particular embodiment the sample may be treated prior to its use, *e.g.* in order to avoid nucleic acids degradation. Techniques of cell lysis, concentration or dilution of nucleic acids are known by the skilled person.

Measuring the expression profile of the miRNA in the sample obtained form the patient can be performed by a variety of techniques.

For example the ribonucleic acids contained in the samples (*e.g.,* cell or tissue prepared from the patient) are first extracted according to standard methods alone or in combination (*e.g.,* lytic enzymes or chemical reagents-based lysis solutions or nucleic-acid-binding resins), following the manufacturer's instructions. The extracted miRNAs is then detected by hybridization (e.g., Northern blot analysis) and / or amplification -e.g., RT-PCR). Real-time quantitative or semi-quantitative RT-PCR is preferred. Real-time quantitative or semi-quantitative RT-PCR is particularly advantageous. Other methods of Amplification include ligase chain reaction (LCR), transcription-mediated amplification (TMA), strand displacement amplification (SDA) and nucleic acid sequence based amplification (NASBA).

In a particular embodiment, the determination comprises hybridizing the sample with selective reagents such as probes or primers and thereby detecting the presence, or measuring the amount of miRNAs originally in the sample. Hybridization may be performed by any suitable device, such as a plate, microtiter dish, test tube, well, glass, column, and so forth In specific embodiments, the hybridization is performed on a substrate coated with the reagent, such as a miRNA array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The hybridization may be made under any condition suitable for a detectable complex, such as a miRNAs hybrid, to be formed between the reagent and the miRNAs of the sample.

Nucleic acids exhibiting sequence complementarity or homology to the miRNAs of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e.g. avidin/biotin).

The probes and primers are "specific" to the miRNAs they hybridize to, *i.e.* they preferably hybridize under high stringency hybridization conditions (corresponding to the highest melting temperature -Tm-, *e.g.,* 50 % formamide, 5x or 6x SCC. 1x SCC is a 0.15 M NaCl, 0.015 M Na-citrate).

Accordingly, the present invention concerns the preparation and use of miRNA arrays or miRNA probe arrays, which are macro arrays or microarrays of nucleic acid molecules (probes) that are fully or nearly complementary or identical to a plurality of miRNA molecules positioned on a support or support material in a spatially separated organization. Macroarrays are typically sheets of nitrocellulose or nylon on which probes have been spotted. Microarrays position the nucleic acid probes more densely such that up to 10,000 nucleic acid molecules can be fit into a region typically 1 to 4 square centimetres. Microarrays can be manufactured by spotting nucleic acid molecules, *e.g.,* genes, oligonucleotides, etc., onto substrates or synthesizing oligonucleotide sequences *in situ* on a substrate. Spotted or *in situ* synthesized nucleic acid molecules can be applied in a high density matrix pattern of up to about 30 non-identical nucleic acid molecules per square centimetre or higher, *e.g.* up to about 100 or even 1000 per square centimetre. Microarrays typically use coated glass as the solid support, in contrast to the nitrocellulose- or nylon- based material of filter arrays. By having an ordered array of miRNA-complementing nucleic acid samples, the position of each miRNA can be tracked and linked to the original sample. A variety of different array devices in which a plurality of distinct nucleic acid probes are stably associated with the surface of a solid support are known to those of skill in the art. Useful substrates for arrays include nylon, glass, metal, plastic, latex, and silicon. Such arrays may vary in a number of different ways, including average probe length, sequence or types of probes, nature of bond between the probe and the array surface, *e.g.* covalent or non-covalent, and the like.

After an array or a set of miRNA probes is prepared and/or the miRNA in the sample or miRNA probe is labeled, the population of target nucleic acids is hybridized with the array or probes under hybridization conditions, where such conditions can be adjusted, as desired, to provide for an optimum level of specificity in view of the particular assay being performed. Suitable hybridization conditions are well known to those of skill in the art and reviewed in Sambrook et al. (2001). Of particular interest in many embodiments is the use of stringent conditions during hybridization. Stringent conditions are known to those of skill in the art.

Alternatively, miRNAs quantification method may be performed by using stem-loop primers for reverse transcription (RT) followed by a real-time TaqMan^{®} probe. Typically, said method comprises a first step wherein the stem-loop primers are annealed to miRNA targets and extended in the presence of reverse transcriptase. Then miRNA-specific forward primer, TaqMan^{®} probe, and reverse primer are used for PCR reactions. Quantitation of miRNAs is estimated based on measured CT values.

Many miRNA quantification assays are commercially available from Qiagen (S.A. Courtaboeuf, France) or Applied Biosystems (Foster City, USA).

Expression profile of a miRNA may be expressed as absolute expression profile or normalized expression profile. Typically, expression profiles are normalized by correcting the absolute expression profile of a miRNA by comparing its expression to the expression of a mRNA that is not a relevant, e.g., a housekeeping mRNA that is constitutively expressed. Suitable mRNA for normalization include housekeeping mRNAs such as the U6, U24, U48 and S18. This normalization allows the comparison of the expression profile in one sample, e.g., a patient sample, to another sample, or between samples from different sources.

The method of the invention further comprises a step that compares the expression profile of said miRNAs in the sample of the patient with a reference profile of said miRNAs, wherein a difference or similarity between said expression profiles is indicative of a Crohn's disease or an ulcerative colitis. The reference profile according to the invention is specific of the disease. The reference profile may thus consist in the expression profile of said miRNAs in a tissue reference such a tissue representative of a Crohn's disease or an ulcerative colitis. Accordingly, the reference profiles may be predetermined by carrying out a method comprising the steps of a) providing at least one collection of tissue samples from Crohn's disease or ulcerative colitis, b) determining for each tissue sample comprised in said collection, the expression profile of said miRNAs.

Typically reference profiles indicative of UC or CD are represented in Table C.

**Table C: reference profiles of UC and CD**

| miRNA | Relative expression in CD | Relative expression in UC |
|---|---|---|
| miR15a | Upregulated (+8) | Upregulated (+7) |
| miR26a | Upregulated (+12) | Upregulated (+9,5) |
| miR29a | Upregulated (+14) | Upregulated (+10) |
| miR29b | Upregulated (+14) | Upregulated (+10) |
| miR30c | Upregulated (+8) | Upregulated (+8) |
| miR126* | Upregulated (+20) | Upregulated (+16) |
| miR127-3p | Upregulated (+11) | Upregulated (+9) |
| miR185 | Upregulated (+10) | Upregulated (+7) |
| miR196a | Upregulated (+16) | Upregulated (+7) |
| miR324-3p | Upregulated (+11) | Upregulated (+10) |
| miR-146b-5p | Downregulated | Non dysregulated |
| miR150 | Upregulated | Downregulated |
| miR-181d | Upregulated | Downregulated |
| miR-182 | Upregulated | Downregulated |
| miR199a-3p | Non dysregulated | Downregulated |
| miR199a-5p | Downregulated (-2) | Downregulated (-10) |
| miR199b-5p | Downregultated | Upregulated |
| miR-203 | Upregultated | Downregulated |
| miR223 | Upregultated (+8) | Downregulated (+2,5) |
| miR-299-5p | Upregulated | Downregulated |
| miR320a | Non Dysregulated | Downregulated |
| miR-146a | Upregultated | Downregulated |
| miR-142-3p | Upregulated | Downregulated |
| miR-142-5p | Upregulated | Downregulated |
| miR-328 | Upregulated | downregulated |

As used herein the term "upregulated" means that the expression of the corresponding miRNA is upregulated in comparison with the expression generally determined in a healthy patient (e.g. a patient not affected with an IBD).

As used herein the term "downregulated" means that the expression of the corresponding miRNA is downregulated in comparison with the expression generally determined in a healthy patient (e.g. a patient not affected with an IBD).

As used herein the term "non dysregulated" means tha the expression of the corresponding miRNA is at the same level than the expression generally determined in a healthy patient (e.g. a patient not affected with an IBD).

Alternatively, the expression profile may be expressed as a score. For example said score may be obtained by i) determining for every miRNA of the profile their expression level in the sample ii) allocating for every miRNA a coefficient (e.g. a positive when upregulated or negative coefficient when down regulated). The advantage of said score is to make easier the comparison step with the reference profiles that may be expressed as "cut-off values". For example the reference ("cut-off") value represents the score calculated for the profile in a tissue sample representative of UC or CD. The cut-off values may also be predetermined by carrying out the method of the invention for tissue sample representative or UC or CD. In particular embodiment, the cut-off values as described above may be reported in a table, so that the physician can compare the score obtained for a patient with said values and can easily determined whether the patient has UC or CD.

A further object of the disclosure relates to a kit for performing the methods of the invention, wherein said kit comprises means for measuring the expression profile of miRNAs in the sample obtained from the patient. The kits may include probes, primers macroarrays or microarrays as above described.

For example, the kit may comprise a set of miRNA probes as above defined, usually made of DNA, and that may be pre-labelled. Alternatively, probes may be unlabelled and the ingredients for labelling may be included in the kit in separate containers. The kit may further comprise hybridization reagents or other suitably packaged reagents and materials needed for the particular hybridization protocol, including solid-phase matrices, if applicable, and standards.

Alternatively the kit may comprise amplification primers (*e.g.* stem-loop primers) that may be pre-labelled or may contain an affinity purification or attachment moiety. The kit may further comprise amplification reagents and also other suitably packaged reagents and materials needed for the particular amplification protocol.

The invention will be further illustrated by the following figures and examples. However, these examples and figures should not be interpreted in any way as limiting the scope of the present invention.

### FIGURES:

**Figure 1****: miRNAs with differentially altered expression in non-inflamed UC and CD tissues : Box-whisker plot analysis.** miRNA expression was measured in non-inflamed colonic mucosa obtained from UC and CD patients (8 patients/IBD) and computed *vs.* that measured in healthy controls. Data corresponding to 6 miRNAs (mir-150, mir-196b, mir-199a-3p, mir-199b-5p, mir-223 and mir-320a) with statistically different alteration of expression in UC and CD mucosal tissues are presented as box-whisker plots (box, 25-75%; whisker, 10-90%; line, median); p<0.05.

### EXAMPLE: miRNA GENE EXPRESSION IS ALTERED IN BOTH UC AND CD COLONIC BIOPSIES:

### Material & Methods:

**IBD patients and controls:** Colonic pinch biopsies were obtained in the course of endoscopical examination of patients with mild to severe CD and UC and of healthy individuals undergoing screening colonoscopies (Table **I**) (Protocol approved by the Local Ethic Committee).

**Table I : Characteristics of patients with CD or UC.**

| | **Ulcerative Colitis** | **Cronh's Disease** |
|---|---|---|
| N° of patients | 8 | 8 |
| Male / Female | 5 / 3 | 4 / 4 |
| Age (* y) | | |
| Mean | 45.9 | 37.6 |
| Range | 33 - 57 | 20 - 58 |
| Disease duration (y) | | |
| Mean | 10.5 | 8.8 |
| Range | 1 - 21 | 0.5 - 23 |
| ^{#} Medications (%) | | |
| 5 ASA | 6 (75) | 2 (25) |
| CS | - | 2 (25) |
| AZA | 1 (13) | 2 (25) |
| MTX, IFX | - | 1 (13) |
| CS, 5 ASA | 1 (13) | - |
| None | - | 1 (13) |

| | | |
|---|---|---|
| * y, years; ^{#} Medications : CS: steroids / 5 ASA: 5 aminosalicylates / AZA: azathioprine / IFX: infliximab / MTX: methotrexate. | | |

Clinical disease activity for CD and UC was assessed according to the Harvey-Bradshaw and to the Colitis Activity (CAI) indexes, respectively. In each IBD patient, endoscopically non-inflamed (quiescent) and inflamed areas of colonic tissue were punctured (5 biopsies/area). Three biopsies from each area were allocated for immediate RNAlater™ immersion, then snap frozen and stored in liquid nitrogen. Two were set apart for histopathological examination. For biopsy collection, quiescent and inflamed areas were separated by more than 20 cm along the colon. In healthy controls, 5 biopsies were punctured both in right and left colon and processed as above. A total of 260 biopsies were processed.

**Histopathological analyses:** Biopsies were routinely stained with hematoxylin and eosin. Histological assessments of mucosal damage and inflammatory cells infiltration were graded by the same expert gastrointestinal pathologist, using a score previously validated to characterize colonic involvement of both UC and CD. Alterations in miRNA gene expression were studied following this histological guideline.

**RNA Isolation:** Total RNAs were extracted from biopsies with TRIzol^{®} reagent (Invitrogen) then quantified using a ND-1000 NanoDrop spectrophotometer (NanoDrop Technologies) and purity/integrity was assessed using disposable RNA chips (Agilent RNA 6000 Nano LabChip kit) and an Agilent 2100 Bioanalyzer (Agilent Technologies, Waldbrunn, Germany). Only RNA preparations with RIN > 7 were further processed for analysis of miRNA expression.

**Reverse-Transcription and Real-Time Q-PCR:** The Human Panel Early Access Kit (TaqMan^{®} MicroRNA Assay; Applied Biosystems) designed to quantify 321 mature human miRNAs was used. cDNA was generated from 10 ng of total RNA using miRNA-specific stem-loop RT primers. Real-Time Q-PCR assays were performed according to the manufacturer's instructions using aliquots of cDNA equivalent to ∼1.3 ng of total RNA and were run in a Light Cycler 480 (Roche Diagnostics).

*Normalization of Real-Time Quantitative PCR results :* Several RNA (U6, U24, U48 and S18) were tested as putative standards and U6 (a ubiquitous small nuclear RNA) was found the most reliable. Expression of miRNAs was thus computed relative to that of U6 and a comparative threshold cycle method (2^{-ΔΔC_{T}}) was used to compare non-inflamed and inflamed IBD tissues with healthy controls.

**Statistical analysis:** Unpaired groups of values were compared according to the non-parametric Mann-Whitney test. Statistical significance was set at p ≤ 0.05..

### Results:

Our objective in investigating alterations of miRNA expression in IBD tissues was to check for specific modifications that may account for early epithelial cell dysfunction in the quiescent colonic mucosa of UC and CD patients (Table I). We thus focused on grades 0 and 1 biopsies, but grades 2-4 (inflamed mucosa) were also studied for comparison of both stages of the disease.
miRNA expression was quantified by Real-Time Q-PCR miRNA assay. Measuring the abundance of 321 human miRNA transcripts (2^{-ΔC_{T}}), preliminary experiments showed that no significant differences were observed between right and left colon in healthy control tissues.
According to our stringent criteria for the selection of miRNA with altered gene expression (10 x Log₁₀2^{-ΔΔC_{T}} > 7 or < -7), up and down regulations were balanced in UC tissues (51.7% and 48.3%, respectively), whereas the vast majority of altered miRNAs (85.4 %) was up-regulated in CD tissues.
*UC tissues:* 173 miRNAs were expressed above the level of detection (C_{T}<35) in UC tissues. Twenty-two miRNAs were differentially expressed when non-inflamed UC and healthy control tissues were compared, 10 and 12 being up-and down- regulated, respectively. Seventeen miRNAs (all but mir-185, 196a, 214, 376a, 424 which did not match our selection criteria) displayed similar dysregulated expression in quiescent and inflamed UC mucosa.
*CD tissues:* 204 miRNAs were expressed above the level of detection in CD tissues. Thirty-four miRNAs were identified as differentially expressed when non-inflamed CD and healthy control tissues were compared, 30 and 4 being up-and down- regulated, respectively. Twenty eight miRNAs (all but mir-9*, 30a*, 30c, 223, 374a, 451 which did not match our selection criteria) displayed similar dysregulated expression in quiescent and inflamed CD mucosa.
Finally, we also noticed alterations in miRNA gene expression specific to inflamed UC or CD tissues (7 and 13 miRNAs, respectively).

*UC and CD tissues:* 10 miRNAs shared common altered expression in non-inflamed UC and CD tissues, of which 7 (all but mir-30c, 185 and 196a) were also overexpressed in both inflamed UC and CD biopsies (Table II).

**Table II : Shared alterations of miRNA gene expression in UC and CD patients**

| **miRNA**_**Id** | **Access**_**N°** | **Relative expression vs. healthy controls** | |
|---|---|---|---|
| | | **Non-inflamed UC** | **Non-inflamed CD** |
| | | **Mean ± Sem** | **Mean ± Sem** |
| hsa-mir-26a | MIMAT0000082 | 9.478 ± 2.917 | 12.881 ± 1.217 |
| hsa-miR-29a | MIMAT0000086 | 10.506 ± 2.428 | 14.166 ± 1.060 |
| hsa-miR-29b | MIMAT0000100 | 10.448 ± 2.176 | 14.671 ± 1.429 |
| hsa-miR-126* | MIMAT0000444 | 16.966 ± 2.750 | 20.311 ± 3.150 |
| hsa-miR-127-3p | MIMAT0000446 | 8.900 ± 2.377 | 11.057 ± 1.259 |
| hsa-mir-15a | MIMAT0000068 | 7.275 ± 2.509 | 8.206 ± 3.514 |
| hsa-miR-324-3p | MIMAT0000762 | 9.929 2.543 | 11.947 ± 1.243 |
| *hsa-mir-30c* | *MIMAT0000244* | *8.389* ± *2.596* | *8.190* ± *1.663* |
| *hsa*-*mir*-*185* | *MIMAT0000455* | *7.325* ± *3.144* | *10.018* ± *4.030* |
| *hsa*-*mir*-*196a* | *MIMAT0000226* | *7.202* ± *4.389* | *16.216* ± *1.205* |

| | | | |
|---|---|---|---|
| Relative miRNA expression was computed *vs.* that measured in healthy controls. miRNA with shared overexpression relative to cut-off values (10 x log₁₀2^{-ΔΔCT} > 7) in both non-inflamed UC and CD tissues are listed. Access_N°, MIMAT identification number; Mean ± Sem (5-8 patients). Italics (3 lower rows), miRNA that are not overexpressed in inflamed UC (mir-185, 196a) or CD (mir-30c). | | | |

Furthermore, 11 miRNAs showed distinct alteration of expression in quiescent UC and CD (Figure 1):

**Table III: miRNA with distinct alterations of gene expression in UC and CD patients**

| **miRNA**_**Id** | **Accession**_**Number** | **UC *vs.* CD** | |
|---|---|---|---|
| | | **p*** | **Number of patients** |
| hsa-miR-146b-5p | MIMAT0002809 | 0.0458 | 8 patients |
| hsa-mir-150 | MIMAT0000451 | 0.0273 | 8 patients |
| hsa-mir-223 | MIMAT0000280 | 0.0357 | 8 patients |
| hsa-mir-181d | MIMAT0002821 | 0.0460 | 8 patients |
| hsa-mir-203 | MIMAT0000264 | 0.0357 | 8 patients |
| hsa-mir-299-5p | MIMAT0002890 | 0.0356 | 8 patients |
| hsa-mir-182 | MIMAT0000259 | 0.0283 | 5 patients |
| hsa-mir-320 | MIMAT0000510 | 0.0163 | 5 patients |
| hsa-mir-199a-3p | MIMAT0000232 | 0.0472 | 5 patients |
| hsa-mir-199a-5p | MIMAT0000231 | 0.0283 | 5 patients |
| hsa-mir-199b-5p | MIMAT0000263 | 0.0283 | 5 patients |

Finally, we analyzed altered miRNA expression in non-inflamed colonic biopsies of CD and UC patients using an unsupervised classification method (ComparativeMarkerSelection; computed on line on the GenePattern server housed on the "Broad Institute" web site; http://www.broadinstitute.org/cancer/software/genepattern/). This allowed the selection of 4 additional miRNAs which help discriminate between CD and UC, although they did not match the abovementioned criteria for miRNA selection (10 x Log₁₀2^{-ΔΔCT} > 7 or < -7) (Table IV).

**Table IV**

| **miRNA**_**Id** | **Accession**_**Number** |
|---|---|
| hsa-mir-146a | MIMAT0000449 |
| hsa-mir-142-3p | MIMAT0000434 |
| hsa-mir-142-5p | MIMAT0000433 |
| hsa-mir-328 | MIMAT0000752 |

When tested using a K Nearest Neighbor Classification with leave-one-out Cross Validation algorithm (KNNXValidation; computed on line on the GenePattern server housed on the "Broad Institute" web site; http://www.broadinstitute.org/cancer/software/genepattern/) the set of 15 miRNA displayed in Tables III and IV allowed the correct classification of 15 out of 16 patients (*see* Table V below).

**Table V**

| Samples | True Class | Predicted Class (KNNXVal) | Correct ? |
|---|---|---|---|
| **CD_Sain_28** | **Cd** | **Uc** | **False** |
| CD_Sain_102 | Cd | Cd | True |
| CD_Sain_111 | Cd | Cd | True |
| CD_Sain_120 | Cd | Cd | True |
| CD_Sain_130 | Cd | Cd | True |
| CD_Sain_137 | Cd | Cd | True |
| CD_Sain_158 | Cd | Cd | True |
| CD_Sain_160 | Cd | Cd | True |
| UC_Sain_107 BI | Uc | Uc | True |
| UC_Sain_125 | Uc | Uc | True |
| UC_Sain_121 | Uc | Uc | True |
| UC_Sain_114 | Uc | Uc | True |
| UC_Sain_109 | Uc | Uc | True |
| **UC_Sain_13** | **Uc later clinically reclassified as CD** | **Cd** | **True** |
| UC_Sain_15 | Uc | Uc | True |
| UC_Sain_132 | Uc | Uc | True |

Altogether, these data unambiguously show that altered miRNA gene expression pre-exists in non-inflamed UC and CD tissues. They support the notion that dysregulated miRNA gene expression may play a key role in the sensitization of quiescent mucosal tissue to inflammation in response to environmental factors or to IBD inducers, thus contributing to the onset and/or relapse of inflammation.

In addition, we anticipate that characterization of altered miRNA expression in pinch colonic biopsies of IBD patients (non-inflamed area) may prove a useful diagnostic tool. The 10 miRNA with common altered overexpression in both CD and UC may help distinguish these patients from healthy individuals. The 15 miRNA displayed in Tables III and IV should prove useful to discriminate between CD and UC patients.

### REFERENCES:

Throughout this application, various references describe the state of the art to which this invention pertains..

## Claims

1. A method for classifying an inflammatory bowel disease in a patient as a Crohn's disease or as an ulcerative colitis, said method comprising:
i) a step of measuring an expression profile of miRNAs in a sample isolated in non-inflamed mucosa of the patient's colon, wherein said miRNAs are miR15a, miR26a, miR29a, miR29b, miR30c, miR126*, miR127-3p, miR-142-3p, miR-142-5p, miR-146a, miR-146b-5p, miR150, miR-181d, miR-182, miR185, miR196a, miR199a-3p, miR199a-5p, miR199b-5p, miR-203, miR223, miR-299-5p, miR320a, miR324-3p, and miR-328 and
ii) a step consisting of comparing the expression profile of said miRNAs in the sample of the patient with control profiles of said miRNAs, wherein a difference or similarity between said expression profiles is indicative of a Crohn's disease or an ulcerative colitis.

2. The method according to claim 1 wherein the sample results from endoscopical biopsies performed in the colon of the patient.

## Patentansprüche

1. Verfahren zur Klassifizierung einer entzündlichen Darmerkrankung bei einem Individuum als Morbus Crohn oder Colitis ulcerosa, wobei das Verfahren umfasst:
i) einen Schritt des Messens eines Expressionsprofils von miRNAs in einer Probe, die aus nicht-entzündeter Schleimhaut des Darms des Individuums isoliert wurde, wobei die miRNAs miR15a, miR26a, miR29a, miR29b, miR30c, miR126*, miR127-3p, miR-142-3p, miR-142-5p, miR-146a, miR-146b-5p, miR150, miR-181d, miR-182, miR185, miR196a, miR199a-3p, miR199a-5p, miR199b-5p, miR-203, miR223, miR-299-5p, miR320a, miR324-3p und miR-328 sind; und
ii) einen Schritt bestehend dem Vergleichen des Expressionsprofils der miRNAs in der Probe des Individuums mit Kontrollprofilen der miRNAs, wobei ein Unterschied oder eine Ähnlichkeit zwischen den Expressionsprofilen Morbus Crohn oder Colitis ulcerosa anzeigt.

2. Verfahren nach Anspruch 1, wobei die Probe aus endoskopischen Biopsien stammt, die im Darm des Individuums durchgeführt wurden.

## Revendications

1. Procédé de classification d'une maladie inflammatoire de l'intestin chez un patient en maladie de Crohn ou en rectocolite hémorragique, ledit procédé comprenant :
i) une étape de mesure d'un profil d'expression de miARN dans un échantillon isolé dans la muqueuse non enflammée du côlon du patient, dans lequel lesdits miARN sont miR15a, miR26a, miR29a, miR29b, miR30c, miR126*, miR127-3p, miR-142-3p, miR-142-5p, miR-146a, miR-146b-5p, miR150, miR-181d, miR-182, miR185, miR196a, miR199a-3p, miR199a-5p, miR199b-5p, miR-203, miR223, miR-299-5p, miR320a, miR324-3p, et miR-328 et
ii) une étape consistant à comparer le profil d'expression desdits miARN dans l'échantillon du patient avec des profils témoins desdits miARN, où une différence ou une similarité entre lesdits profils d'expression indique une maladie de Crohn ou une rectocolite hémorragique.

2. Procédé selon la revendication 1, dans lequel l'échantillon résulte de biopsies endoscopiques réalisées dans le côlon du patient.


### SEQUENCE LISTING

<110> INSERM
<120> A METHOD FOR CLASSIFYING AN INFLAMMATORY BOWEL DISEASE AS A CROHN'S DISEASE OR AS AN ULCERATIVE COLITIS
<130> BIO08361 OGIERDENIS/MC
<160> 25
<170> PatentIn version 3.3
<210> 1
   <211> 22
   <212> RNA
   <213> Homo sapiens
<400> 1
   uagcagcaca uaaugguuug ug 22
<210> 2
   <211> 22
   <212> RNA
   <213> Homo sapiens
<400> 2
   uucaaguaau ccaggauagg cu 22
<210> 3
   <211> 22
   <212> RNA
   <213> Homo sapiens
<400> 3
   uagcaccauc ugaaaucggu ua 22
<210> 4
   <211> 23
   <212> RNA
   <213> Homo sapiens
<400> 4
   uagcaccauu ugaaaucagu guu 23
<210> 5
   <211> 23
   <212> RNA
   <213> Homo sapiens
<400> 5
   uguaaacauc cuacacucuc agc 23
<210> 6
   <211> 21
   <212> RNA
   <213> Homo sapiens
<400> 6
   cauuauuacu uuugguacgc g 21
<210> 7
   <211> 22
   <212> RNA
   <213> Homo sapiens
<400> 7
   ucggauccgu cugagcuugg cu 22
<210> 8
   <211> 22
   <212> RNA
   <213> Homo sapiens
<400> 8
   ugagaacuga auuccauggg uu 22
<210> 9
   <211> 22
   <212> RNA
   <213> Homo sapiens
<400> 9
   ucucccaacc cuuguaccag ug 22
<210> 10
   <211> 21
   <212> RNA
   <213> Homo sapiens
<400> 10
   cauaaaguag aaagcacuac u 21
<210> 11
   <211> 23
   <212> RNA
   <213> Homo sapiens
<400> 11
   uguaguguuu ccuacuuuau gga 23
<210> 12
   <211> 22
   <212> RNA
   <213> Homo sapiens
<400> 12
   ugagaacuga auuccauagg cu 22
<210> 13
   <211> 23
   <212> RNA
   <213> Homo sapiens
<400> 13
   aacauucauu guugucggug ggu 23
<210> 14
   <211> 24
   <212> RNA
   <213> Homo sapiens
<400> 14
   uuuggcaaug guagaacuca cacu 24
<210> 15
   <211> 22
   <212> RNA
   <213> Homo sapiens
<400> 15
   uggagagaaa ggcaguuccu ga 22
<210> 16
   <211> 22
   <212> RNA
   <213> Homo sapiens
<400> 16
   uagguaguuu cauguuguug gg 22
<210> 17
   <211> 22
   <212> RNA
   <213> Homo sapiens
<400> 17
   acaguagucu gcacauuggu ua 22
<210> 18
   <211> 23
   <212> RNA
   <213> Homo sapiens
<400> 18
   cccaguguuc agacuaccug uuc 23
<210> 19
   <211> 23
   <212> RNA
   <213> Homo sapiens
<400> 19
   cccaguguuu agacuaucug uuc 23
<210> 20
   <211> 22
   <212> RNA
   <213> Homo sapiens
<400> 20
   gugaaauguu uaggaccacu ag 22
<210> 21
   <211> 22
   <212> RNA
   <213> Homo sapiens
<400> 21
   ugucaguuug ucaaauaccc ca 22
<210> 22
   <211> 22
   <212> RNA
   <213> Homo sapiens
<400> 22
   ugguuuaccg ucccacauac au 22
<210> 23
   <211> 22
   <212> RNA
   <213> Homo sapiens
<400> 23
   aaaagcuggg uugagagggc ga 22
<210> 24
   <211> 20
   <212> RNA
   <213> Homo sapiens
<400> 24
   acugccccag gugcugcugg 20
<210> 25
   <211> 22
   <212> RNA
   <213> Homo sapiens
<400> 25
   cuggcccucu cugcccuucc gu 22